# EUROPEAN PATENT SPECIFICATION

(11) **EP 1 578 357 B1**
(45) Date of publication and mention of the grant of the patent: **17.02.2010**
(21) Application number: 03745632.4
(22) Date of filing: 27.03.2003
(51) Int. Cl.: C12Q 1/68, C07H 21/04

(54) **METHODS AND COMPOSITIONS FOR ANALYSIS OF URINE SAMPLES IN THE DIAGNOSIS AND TREATMENT OF KIDNEY DISEASES**
VERFAHREN UND ZUSAMMENSETZUNGEN ZUR ANALYSE VON URINPROBEN BEI DER DIAGNOSE UND BEHANDLUNG VON NIERENERKRANKUNGEN
TECHNIQUES ET COMPOSITIONS DESTINEES A L'ANALYSE D'ECHANTILLONS D'URINE EN VUE DU DIAGNOSTIC ET DU TRAITEMENT DE MALADIE RENALES

(30) Priority: 28.03.2002 US 108969
(43) Date of publication of application: 28.09.2005
(73) Proprietor: THE REGENTS OF THE UNIVERSITY OF MICHIGAN, Ann Arbor, Michigan 48109-1280 (US)
(72) Inventor: KURNIT, David, M., Ann Arbour, MI 48103 (US)
(74) Representative: Nash, David Allan
(86) International application number: PCT/US2003/009389
(87) International publication number: WO 2003/082202

(56) References cited:
- WO-A-00/77044
- WO-A-99/47562
- AALTONEN P ET AL: "Changes in the expression of nephrin gene and protein in experimental diabetic nephropathy." LABORATORY INVESTIGATION; A JOURNAL OF TECHNICAL METHODS AND PATHOLOGY SEP 2001, vol. 81, no. 9, September 2001 (2001-09), pages 1185-1190, XP002444159 ISSN: 0023-6837
- NAKAMURA T ET AL: "URINARY EXCRETION OF PODOCYTES IN PATIENTS WITH DIABETIC NEPHROPATHY" NEPHROLOGY DIALYSIS TRANSPLANTATION, OXFORD UNIVERSITY PRESS, GB, vol. 15, no. 9, September 2000 (2000-09), pages 1379-1383, XP009065526 ISSN: 0931-0509
- KIM Y-H ET AL: "Podocyte depletion and glomerulosclerosis have a direct relationship in the PAN-treated rat" KIDNEY INTERNATIONAL, NEW YORK, NY, US, vol. 60, no. 3, September 2001 (2001-09), pages 957-968, XP002995525 ISSN: 0085-2538
- HARA M ET AL: "URINARY EXCRETION OF PODOCYTES REFLECTS DISEASE ACTIVITY IN CHILDREN WITH GLOMERULONEPHRITIS" AMERICAN JOURNAL OF NEPHROLOGY, KARGER, BASEL, CH, vol. 18, no. 1, January 1998 (1998-01), pages 35-41, XP009065519 ISSN: 0250-8095
- KESTILA M ET AL: "Positionally cloned gene for a novel glomerular protein (nephrin) is mutated in congenital nephrotic syndrome" MOLECULAR CELL, CELL PRESS, CAMBRIDGE, MA, US, vol. 1, no. 4, March 1998 (1998-03), pages 575-582, XP002109298 ISSN: 1097-2765
- NAKAMURA T ET AL: "The urinary podocyte as a marker for the differential diagnosis of idiopathic focal glomerulosclerosis and minimal-change nephrotic syndrome." AMERICAN JOURNAL OF NEPHROLOGY 2000 MAY-JUN, vol. 20, no. 3, May 2000 (2000-05), pages 175-179, XP002444160 ISSN: 0250-8095
- DATABASE GENBANK [Online] KOBUNE M. ET AL: 'Indian hedgehog gene transfer augments hematopoietic support of human stromal cells including NOD/SCID-beta2m-/- repopulating cells', XP002995520 Retrieved from NCBI Database accession no. (NM_002181) & BLOOD vol. 104, no. 4, 2004, pages 1002 - 1009
- CARPENTER D. ET AL: 'Characterization of two patched receptors for the vertebrate hedgehog protein family' PROC. NATL. ACAD. SCI. vol. 95, no. 23, 10 November 1998, pages 13630 - 13634, XP002101428
- EISENBERGER C.F. ET AL: 'Diagnosis of renal cancer by molecular urinalysis' JOURNAL OF THE NATIONAL CANCER INSTITUTE vol. 91, no. 23, 01 December 1999, pages 2028 - 2032, XP002995521
- LUIMULA P. ET AL: 'Alternatively Spliced Nephrin in Experimental GLomerular Disease of the Rat' PEDIATRIC RESEARCH vol. 48, no. 6, December 2000, pages 759 - 762, XP002995522
- CHIANG P.W. ET AL: 'Molecular Analysis of Urine Sediment for Follow-up of Urinary Tract Cancers' JOURNAL OF THE NATIONAL CANCER INSTITUTE vol. 92, no. 21, 01 November 2000, pages 1779 - 1780, XP002995523
- KRIZ W. ET AL: 'Progression of glomerular diseases: Is the podocyte the culprit?' KIDNEY INTERNATIONAL vol. 54, 1998, pages 687 - 697, XP002995524
- KIM Y.H. ET AL: 'Podocyte depletion and glomerulosclerosis have a direct relationship in the PAN-treated rat' KIDNEY INTERNATIONAL vol. 60, no. 3, September 2001, pages 957 - 968, XP002995525
- BOTEZATU I. ET AL: 'Genetic Analysis of DNA Excreted in Urine: A New Approach for Detecting Specific Genomic DNA Sequence from Cells Dying in an Organism' CLINICAL CHEMISTRY vol. 46, no. 8, August 2000, pages 1078 - 1084, XP001080049
- LUIMULA P. ET AL: 'Nephrin in experimental glomerular disease' KIDNEY INTERNATIONAL vol. 58, no. 4, October 2000, pages 1461 - 1468, XP002995526
- DATABASE GENBANK [Online] 25 May 2001 STRAUSBERG R., XP002995527 Retrieved from NCBI Database accession no. (BC008633)

## Description

### FIELD OF THE INVENTION

The invention relates to the analysis of biological samples in the diagnosis and treatment of kidney disease. Specifically, the invention relates to the analysis of urine samples to diagnose as well as to monitor the progress of treatment of kidney diseases and disorders associated with a variety of conditions including, but not limited to, glomerulonephritis, nephrotic syndrome, diabetes, lupus, hypertension, acute tubular necrosis (ATN), renal obstructive disorders, renal cancers and other diseases or syndromes.

### BACKGROUND OF THE INVENTION

Physicians who treat patients often require analyses of kidney status and function. For diseases such as glomerular disorders, this is currently accomplished by detection of protein (albumin) present in the urine, that is, by assaying the amount of protein spilled into the urine from the kidney. However, this method can be insensitive and untimely because much kidney function must be lost before an abnormal test result appears. It may also be inaccurate because there are many non-specific causes of protein in the urine, and testing for elevated albuminuria may be falsely positive in many subjects. Moreover, for some kidney disorders, such as proximal tubule disorders, sensitive urine-based tests are generally not available. The alternative for analysis is a kidney biopsy that is expensive, invasive and associated with a finite morbidity due to the risks of bleeding and infection.

The only currently available urine-based test for monitoring podocyte disease directly, which is used only experimentally, is an antibody test for podocytes excreted in the urine. However, this test is not as sensitive as the method we present, as it only becomes abnormal when albumin is present in the urine. Thus, there is need for a specific, generally available test that shows positive association with renal dysfunction earlier in the course of kidney disease, preferably close to the outset.
KIM Y-H ET AL KIDNEY INTERNATIONAL, NEW YORK, NY, US, vol. 60, no. 3, September 2001 (2001-09), pages 957-968, XP002995525 ISSN: 0085-2538; discloses a method for detecting a kidney glomerular disorder by screening rat urine for the presence of podocyte marker GLEPP1 mRNA. Puromycin aminonucleoside (PAN) treated rats are used as a model for glomerulosclerosis. PAN causes a reduction in glomerular podocyte counts, and the loss of podocytes is, among other parameters, evaluated by determining the presence of GLEPP1 mRNA in urine. The detection of nephrin mRNA is also mentioned but no experimental data are shown.

The invention addresses this situation with novel indicators of kidney status or function that permit identification of abnormal desquamation of a given cell type by expression of a gene unique to that cell in the urinary tract.

Thus, there is need for these sensitive methods and compositions that assess renal status accurately both earlier and later in the course of kidney disease than is feasible with other urine-based tests.

### SUMMARY OF THE INVENTION

In accordance with the invention, novel compositions, systems, and methods are provided to diagnose and manage kidney-related diseases and disorders and related indicators. For purposes of the invention, the terms "kidney disease(s)" and "kidney disorder(s)" are interchangeable and mean any disease, disorder, syndrome, anomaly, pathology, or abnormal condition of the kidney or of the structure or function of its constituent parts. In accordance with the invention, there has now been discovered a methodology for uniquely identifying particular genes expressed only in urinary tract cells of particular clinical or scientific interest and specifically in identifying and monitoring the treatment of renal disease. By detection of kidney cells (podocytes) that are not normally present in urine, but are present when renal conditions are present that affect these cells, detection of these conditions is possible.

In one embodiment, unique methods and compositions allow detection of the presence of glomerular podocytes and/or proximal tubule cells to assess onset of renal conditions, as well as to monitor the response of podocytes to therapy.

In accordance with the invention, the presence of these cells is detected by screening for expression of a gene that occurs only in the particular cell of interest in the urinary tract (podocyte). In other embodiments, the invention comprises the analysis of genes expressed in urine sediment cells and provides non-invasive, rapid and accurate analysis of kidney status and function in many medical diseases and disorders.

In still further embodiments, the invention comprises the analysis of gene expression in cells exfoliated spontaneously into urine in order to diagnose such disorders and syndromes rapidly using non-invasive urine-based tests. In one embodiment, detection of gene expression uses a reverse transcriptase-quantitative polymerase chain reaction (RT-QPCR) to uniquely detect podocytes (by expression of nephrin, expressed uniquely in these cells in the urinary tract).
In accordance with the instant invention, these indicators become positive earlier in the course of disease than albuminuria and are more specific. In further embodiments the invention comprises novel primers and probes, and kits containing same, for the detection of gene expression in cells of interests.

### BRIEF DESCRIPTION OF THE DRAWINGS

Figure 1a and 1b show one embodiment of steps in *TaqMan* assay.

Figure 2 shows one embodiment of steps in the *AmpliSensor* assay.

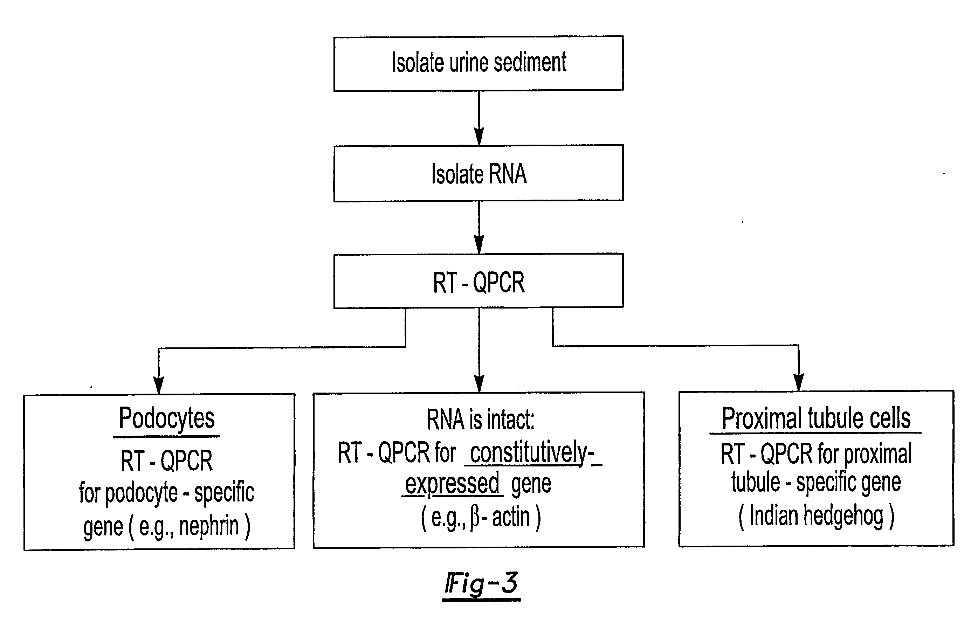

Figure 3 is a flow chart of steps in one embodiment, without limitation, of the present invention.

### DETAILED DESCRIPTION OF PREFERRED EMBODIMENTS

In one embodiment, without limitation, the invention comprises the identification and analysis of one or more specific gene sequences to mark podocytes and one or more other specific gene sequences to mark proximal tubule cells. These two cell types are known to be affected by many kidney disorders, by way of examples only, the listed disorders. In the invention, genes expressed in these cell types alone are identified in order to mitigate confounding results due to gene expression in other cell types, such as lower urinary tract cells, that are exfoliated normally into urine.

The identification of such genes dictates the use of sensitive methodologies. For example, using less sensitive methods, genes thought not to be expressed in certain cells were in fact expressed at low levels (e.g., 1% of the level in cells known to express the gene). Since most cells in the urine sediment originated from lower urinary tract cells, a low level of transcription in these cells interfered with sensitive RT-QPCR studies to detect upper urinary tract cells. In contrast, in accordance with the instant invention, unique identification of genes expressed solely in upper urinary tract cells of interest, by way of example only and without limitation, nephrin in podocytes, is possible. For example, in the latter stages of disease, albuminuria can be fixedly abnormal and thus unresponsive to therapy designed to prevent podocyte loss. At this time, if therapy is effective at preventing podocyturia, nephrin RT-QPCR can be normal when the albuminuria test is fixedly abnormal due to pre-existing damage to podocytes that cannot be repaired.

The invention also comprises identification of exfoliation of the cells of interest in particular disorders. We have shown unexpectedly in the invention that normal kidneys do not release podocytes. However, disorders or diseases involving these cells result in the loss of podocytes into urine sediment. This means that at least a fraction of the released cells are present as intact cells or fragments of cells that can be spun down into the urine sediment. The invention identifies this exfoliation for purposes of assessment, diagnosis and treatment.

In accordance with another aspect of the instant invention, specific probes are provided that differ from any of those found in existing art. Although previous expression analyses delineated potential probe candidates, none used a technique as sensitive as RT-QPCR, and thus none could identify conclusively what an appropriate marker would be. For example, glepp1 showed promise as a candidate for a podocyte-specific marker based on less sensitive expression studies that showed it to be expressed only in the podocyte in the urinary tract. However, the RT-QPCR techniques of the present invention showed a low, previously undetected, amount of glepp1 transcription in lower urinary tract cells, in addition to the larger expected synthesis in podocytes. Thus, expression of the glepp1 gene was not suitable as a marker for podocytes. This resulted from confounding by this low, but finite, amount of gene expression in lower urinary tract cells. In contrast, use of the invention showed that nephrin was not expressed in urinary tract cells, other than podocytes, so that its presence in urine sediment could be used as a surrogate marker for podocyturia.

Similarly, many potential markers for tubule cells were present in normal urine sediments, reflecting expression of these genes in small amounts in lower urinary tract cells. This was true even when screening candidate genes (such as N-acetyl-β-D-glucosaminidase, aquaporin 1, uromodulin and SLC3A1) shown by less sensitive analyses to be expressed in only a particular region of the kidney.

In one embodiment, without limitation, the invention also comprises the analysis by RT-QPCR of extant ribonucleic acid ("RNA") in urine sediment to identify cells of interest. This requires that the RNA in at least a portion of the cells exfoliated into urine sediment is sufficiently intact to generate RT-QPCR product, a result not appreciated heretofore. Thus, for purposes of the invention, the cells of interest may be considered whole cells or fragments thereof sufficient to provide intact RNA. The invention also comprises the expression of a ubiquitously transcribed housekeeping gene to confirm that RNA capable of priming reverse transcription has been isolated.

In a preferred embodiment, without limitation, the invention comprises the use of RT-QPCR technology to detect the abnormal presence of podocytes in the urine sediment in disorders involving these kidney cells, respectively. However, one of ordinary skill will appreciate that there are other embodiments of the invention that may use other techniques to detect expression of relevant genes. Thus, using the methodology of the instant invention, it is clinically possible to separate patients into those who are desquamating podocytes into the urine, and those who are not.

Further, the RT-QPCR test of the invention yields a semi-quantitative analysis of renal involvement. This affords the possibility of utilizing molecular biomarkers to monitor the progression of renal disorders or diseases using only non-invasively obtained urine. The abnormalities are potentially determined much earlier than by using markers of elevated albuminuria or abnormal renal function.

In accordance with the invention, sensitive tests using RT-QPCR for nephrin (podocytes) require only a small amount of urine; are non-invasive as they only require spontaneously voided urine; and reflect the current status of kidney involvement. This provides the clinician with an opportunity to monitor the effects of therapy. For example, diabetic subjects who are fixedly albuminuria but who are negative by nephrin RT-QPCR are uniformly later-stage patients on therapy (ACE inhibitors or angiotensin 2 receptor antagonists). Using the invention early in the course of disease, the nephrin RT-QPCR changes seen can antedate the later changes of elevated albuminuria and clinically-apparent nephropathy. Later in the course of disease, nephrin RT-QPCR sensitively detects individuals in whom drug therapy is successfully preventing the loss of podocytes. As such, nephrin RT-QPCR represents a sensitive way both to detect nephrologic anomalies and to monitor their therapy.

Embodiments of the invention also comprise use of the RT-QPCR method to detect kidney damage as assayed by the presence of a specific type of injured kidney cell in urine sediment. Thus, the invention circumvents both the sensitivity difficulties and inexactitudes of detecting albumin in the urine using the previous art. Although Hara and coworkers have developed an immunological test to detect podocytes in the urine, the test is limited because it only detects abnormalities in diabetic subjects and pediatric subjects who already have elevated albuminuria. In contrast, the invention comprises a more sensitive assay that, as described herein, shows abnormalities in nephrin RT-QPCR (podocytes) earlier than the immunologic test.

To detect many kidney diseases, including, as one example only, glomerular diseases, some embodiments of the invention are able to utilize the gene, nephrin, whose transcription in the urinary tract is limited to podocytes. In other embodiments, any gene whose transcription in the urinary tract is limited to podocytes may be used for this purpose. Abnormal states showing the presence of podocytes in urine sediment include, as only some examples, glomerulopathies, nephrotic syndrome, systemic lupus erythematosus (SLE), diabetes and hypertension. These nephrin RT-QPCR changes are not seen in non-glomerular renal disorders that do not result in shedding of podocytes. The nephrin RT-QPCR test results obtained by use of the invention show abnormal levels before other urinary markers of renal injury. Later in the course of disease, elevated albuminuria is present with or without RT-QPCR anomalies. At such a point, a negative nephrin RT-QPCR test may indicate that there is effective therapy so that podocytes do not continue to be lost even in the presence of elevated albuminuria that reflects previous podocyte loss.

In some embodiments, the invention also comprises simple preservation protocols after which the RNA from treated urine sediment cells is stable (and can be mailed) at room temperature. The urine sample is obtained non-invasively and preserved simply at the point of care. It is then shipped in a small volume at ambient temperature to a centralized facility where a sophisticated RT-QPCR analysis is performed. The results can be obtained within a day following receipt of the sample and reported back to the referring physician.

**EXAMPLES:**

**Isolation of RNA.** Informed consent was obtained for acquisition of all urine samples. The sample consisted of *ca.* 3-40 ml of spontaneously voided urine. Urine used for this purpose was fresh or frozen previously. Urine was spun down for 10 min at 8,000 rpm. The supernatant was decanted, the pellet was washed once in phosphate buffered saline, pH 7.4 (137 mM NaCl + 2.7 mM KCl + 4.3 mM Na₂HPO₄ + 1.4 mM KH₂PO₄) spun down again and the air-dried pellet was resuspended in a small volume of double distilled water, aliquoted and frozen at -80° C.

Using the invention, RNA in urine sediment cells was detected. In the body, urine is stored in the bladder for hours at approximately 37°C before it is excreted. The urine is often then left out for several hours in the clinic at room temperature. Then the urine sediment is spun down and treated with RNAlater (Ambion) or STG buffer (Biotronics, Lowell MA) to inactivate RNase activities. The urine RNA was uniformly sufficiently intact to direct RT-QPCR when samples were obtained within 12 hours of spontaneous voiding. Incubating the urine at room temperature for several days demonstrates that a significant percentage of samples have RNA degradation marked by an inability to support RT-QPCR of the reference β-action marker. This explains the need to treat samples shortly after clinic before the RNA degrades to the point where it is no longer sufficiently large to direct RT-QPCR. RNA is then isolated by means known in the art, for example, following the RNA isolation procedure of Biotronics using its urine sediment RNA isolation kit.

**Primers and probes.**

The primers and probe for human nephrin used in the *AmpliSensor* reaction of some embodiments comprise the following:

Excess primer: 5'-GCCCCGAGGGTCCTGAAGGTG-3' (located at junction of exon 23 and exon 24)(SEQ ID NO: 1);

Limiting primer: 5'-TGGACTGGCTGACAAAGGGACCCAG-3' (located in exon 22) (SEQ ID NO: 2); and

*AnapliSensor probe:* 5'-TACTGGAGAACCTGAAGACCAGCTGCC-3' (located in exon 23) (SEQ ID NO: 3).

The primers and probe for human β-actin used in the *TaqMan* reaction of some embodiments comprise the following:
β-actin-forward primer: 5'-AACTTGAGATGTATGAAGGCTTTTGG-3' (SEQ ID NO: 7);
β-actin-reverse primer: 5'- TTTTTTTTTTTTTAAG-3' (SEQ ID NO: 8); and
β-actin-*TaqMan* probe: 5'-CAACTGGTCTCAAGTCAGTGTACAGGTAAGCCCT-3' (SEQ ID NO: 9), in one embodiment, among others, labeled as
6FAM-5'-CAACTGGTCTCAAGTCAGTGTACAGGTAAGCCCT-3'-TAMRA (labeled SEQ ID NO: 9).

In other aspects of the invention, kits can be provided where one or more of the disclosed novel primers or probes are provided, in a container or otherwise, for use in detecting cells of interest by the inventive methods disclosed herein.

In some embodiments, without limitation, the invention comprises a PCR process disclosed in U.S. Patent Nos. 4,965,188 and 5,079,352 and/or the *TaqMan* RT-QPCR methodology disclosed in U.S. Patent No. 5,210,015.
In some embodiments, without limitation, the invention comprises a PCR process disclosed in U.S. Patent Nos. 4,965,188 and 5,079,352 and/or the *AmpliSensor* RT-QPCR methodology disclosed in U.S. Patent Nos. 5,348,853 and 5,567,583.

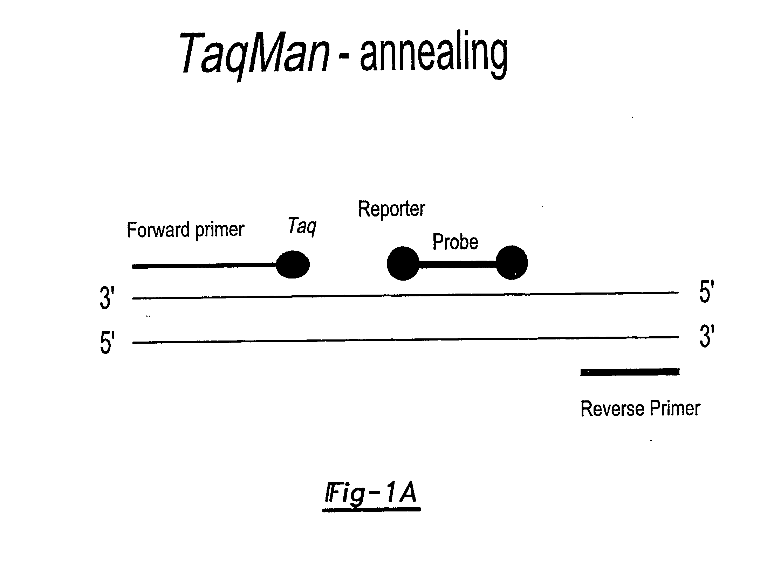

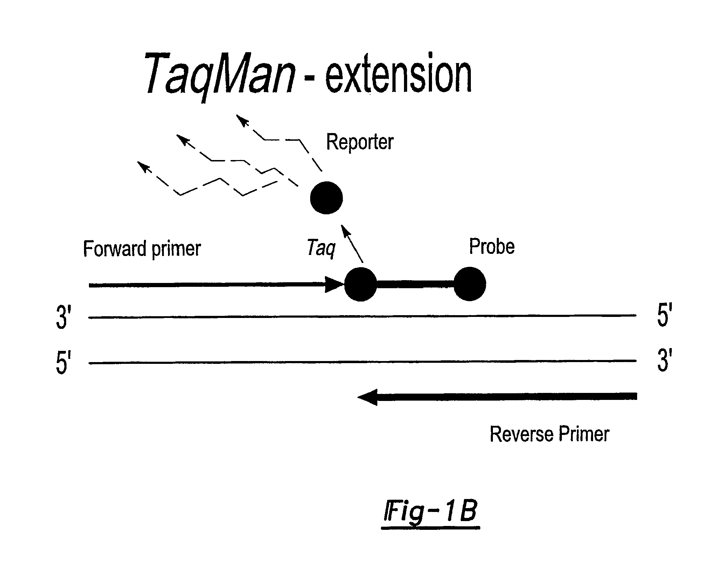

**Principle of *TagMan* assay.** The *TaqMan* assay has been described and is known to those of ordinary skill in the art (see Figures 1A and 1B). The assay is based on the principle that successful polymerase chain reaction (PCR) yields a fluorescent signal due to Taq-mediated exonuclease digestion of a fluorescently-labeled oligonucleotide homologous to a sequence between the two primers. The extent of digestion, which depends directly on the amount of PCR that occurs, can be quantified directly and accurately by measuring the increment in fluorescence that results from decreased energy transfer. This measurement allows detection in the exponential phase of the PCR reaction that is required for determination of initial genomic sequence copy number.

**Principle of *AmpliSensor* assay**. To measure the desquamation of podocytes into urine in a variety of disorders, the *TaqMan* system may not be sufficiently sensitive or specific, and for this demanding case, the *AmpliSensor* assay was used.

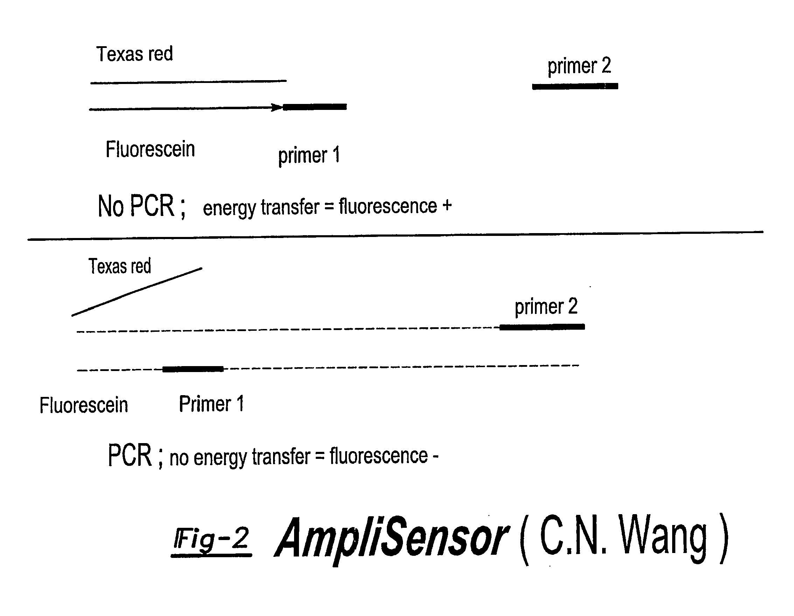

The *AmpliSensor* assay (Figure 2) is based on the principle that decrement of fluorescence energy transfer can be used to measure PCR-mediated disruption of an oligonucleotide duplex, the *"AmpliSensor".* The two strands of the oligonucleotide duplex are modified with donor and acceptor fluorophores, respectively. In the native duplex state that results from the absence of PCR, energy transfer between the fluorophores (the donor, fluorescein, and the receptor, Texas Red) is efficient; the fluorescent signal at the emission frequency of Texas Red following stimulation at the excitation frequency for fluorescein is high. PCR disrupts this oligonucleotide duplex due to strand displacement, decreasing energy transfer (which varies inversely with the sixth power of the distance between the 2 fluors). The extent of disruption, which depends directly on the amount of PCR that occurs, can be quantified directly and accurately by measuring the decrement in fluorescence that results from decreased energy transfer. The *AmpliSensor* system is inherently more sensitive than the *TaqMan* system as the bipartite *AmpliSensor* primer and probe sequence is a single molecule that participates actively in the polymerase chain reaction. By comparison, the probe sequence in *TaqMan* relies passively on hybridization for an energy-transfer-mediated fluorescent event to occur. As a result, the *AmpliSensor* system is more sensitive than the *TaqMan* system.

**Housekeeping gene probe that reacts only with RNA.** A probe for the constitutively-expressed gene, β-actin, was used to verify that RNA was prepared successfully from urine sediment. Although probes have been synthesized to recognize spliced sequences that characterize transcribed sequences, these probes do not distinguish true genes from processed pseudogenes. As a result, genomic DNA contamination that frequently exists in rapid RNA extractions can yield false positives for constitutively expressed genes that have processed pseudogenes. To detect only the truly transcribed sequences, we used the poly(A) cDNA-specific method where one of the primers consisted mainly of a stretch of T residues. This stretch of T residues hybridizes with the polyadenylated tail specific to transcripts. Three nucleotides at the 3' end of the oligonucleotide are complementary to the message to prevent stuttering of the primer on the poly A tail. This set reacted with negligible efficiency to genomic DNA (≤ 0.1% of the signal achieved with total RNA) and could therefore be used to distinguish transcribed sequences from genomic DNA.

**A. Podocytes**

In the absence of a kidney biopsy, early diagnosis of kidney disease resulting from loss of podocytes is usually done indirectly on the basis of elevated excretion of protein (or albumin) into the urine. However, this test is not specific as there are many factors that can affect the excretion of albumin into the urine. Further, abnormalities of this test require a significant loss of podocytes to the point where flattening of the remaining podocytes is inadequate to yield a normal barrier, resulting in leakage of protein into the urine. Since podocytes do not replicate after maximal size of the kidney is achieved, it is especially important to detect the loss of such cells early to prevent loss of kidney function.

In accordance with one aspect of the invention, podocyte loss is detected with a high degree of sensitivity by the abnormal presence in urine sediment of a gene selectively expressed in the podocyte so as to be podocyte-specific in the urinary tract. To illustrate this, we used the gene, nephrin, whose transcription in the urinary tract occurs only in podocytes. The resulting invention comprises a rapid and non-invasive method to screen for early effects on the kidney of disorders that affect the podocytes.

The sensitivity of nephrin detection by RT-QPCR was compared with the presence of protein (albumin) in the urine at defined times after the onset of the chronic disease, diabetes. The sensitivity of this assay for nephrin was also compared with an antibody test for the presence of the podocyte specific-marker, podocalyxin.

**Nephrin marker for podocytes.** There are multiple genes whose expression in the urinary tract was thought to be podocyte-specific. Utilizing such sequences, *TaqMan* RT-QPCR analyses were used with both glepp1 and nephrin. Multiple probes were constructed and these gave either no signal or gave background in normal samples. However, specific signals were not obtained, reflecting the very low abundance of these genes in total urine sediment RNA, as the podocyte constitutes only a small fraction of the cells in urine sediment even in the case of glomerular disease.

Examination of glepp1 with the more sensitive *AmpliSensor* system yielded the unexpected result that normal individuals showed discrete amounts of glepp1 in their urine. Using a rat *TaqMan* gleppl probe on RNA from specific parts of the rat urinary tissue, a low, but finite, level of transcription of glepp1 was detected in bladder cells. Although this level of transcription was too low to be detected by most previous analyses (*ca*. 1% of the level in the glomerulus), it interfered with sensitive RT-QPCR analyses of urine sediment as the majority of exfoliated cells originated from the bladder.

The next candidate probe for selective isolation in the urinary tract to the podocyte tried was nephrin. Nephrin is a structural protein in podocytes, comprising a molecule used to zipper the slit membrane that separates podocytes. Using a rat *TaqMan* probe on RNA from specific parts of the rat urinary tissue, transcription of nephrin was observed only in the glomerulus. In addition to this rat anatomic study, the human nephrin probe was constructed to avoid any chance of cross-reaction with genomic sequences, using the fact that exons 22 and 23, as well as exons 23 and 24, are each separated by over 3 kb of intronic sequences. The *AmpliSensor* set spanned over these 3 exons and showed no detectable reaction with human genomic DNA. Thus, in some embodiments, an *AmpliSensor* nephrin probe was constructed for the invention that was both very sensitive and specific.

Table 1 shows that this nephrin probe does not yield false positives. Both intra-plate and inter-plate assays show that nephrin mRNA is not present in urine sediment from normal individuals. In addition, examination of over 200 different normal urine sediments from 40 normal subjects showed no positives > 2E+00 (= 2 x 10° = 2) molecules of nephrin mRNA/ urine sediment assay (corresponding to sediment from 5 ml of urine).

The potential for false negatives due to degradation of RNA was also evaluated. RNA preparation with the β-actin probe was routinely monitored to ensure that RNA of adequate quality was prepared. This quality control was necessary but did not show whether there was either specific degradation of nephrin mRNA or de-differentiation of podocytes such that they no longer expressed nephrin. Such de-differentiation for glepp1. has been demonstrated to occur in the podocytes of glomeruli undergoing focal segmental glomerulosclerosis, minimal change nephropathy or congenital nephrosis. However, the presence of nephrin by the RT-QPCR test of some- embodiments in all these disorders makes it unlikely that podocytes not expressing nephrin represent a significant problem for the technique.

We found that podocytes are not secreted normally into urine sediment. Thus, an absolute number of nephrin mRNA > 5/urine sediment aliquot may be established as abnormal. Normal samples did not have values > 2, so a value of 5 was used as a conservative breah-point knowing that there are many unknowns relating to renal volume and the ability of the kidneys to concentrate urine. Although this does not account for the concentrating abilities of the kidneys, normalization is difficult to accomplish since there is no way to normalize for the presence of a small number of cells, the podocytes, that are not present normally in urine sediment. Indeed, if the shedding of podocytes varies only with time, normalization to a urine concentration marker (such as creatinine) would not be appropriate. Fortunately, virtually any presence of nephrin is abnormal.

However, since normalization to creatinine is commonly done, nephrin RT-QPCR values were also normalized by dividing by the creatinine concentration obtained in 96 samples from an aliquot of the same urine upon which nephrin RT-QPCR was performed. After such normalization, only 2/96 samples diverged from the original diagnosis (using the empirically derived cut-off point as 5 x 10⁻² dl/mg between normal and abnormal values of nephrin RT-QPCR/urinary creatinine). Further, as expected, both of the samples that diverged using the two types of analysis had borderline values of RT-QPCR. The creatinine concentration rarely varied by more than an order of magnitude, whereas the absolute value for nephrin RT-QPCR varied over many orders of magnitude. Thus, the *AmpliSensor* technique of some embodiments shows excellent specificity regardless of whether the data is normalized to creatinine.

**Comparison of the RT-QPCR analysis, u-podocytes and elevated albuminuria in pediatric nephrologic disorders.** Table 2 illustrates that nephrin RT-QPCR, u-podocyte analysis for podocalyxin and the presence of albumin in the urine are all discordant. Analyses of u-podocytes and albumin in the urine were performed by Dr. M. Hara in Japan. In addition to these analyses, a portion of the urine sediment was treated with RNAlater (Qiagen). RNAlater-treated samples were sent at ambient temperature from Japan to Michigan. The urine sediment was harvested in Michigan by spinning down the RNAlater-treated cells, washing once with phosphate buffered saline, and isolating RNA by the Biotronics protocol *(vide supra).* As shown by *TaqMan* RT-QPCR analyses with the β-actin probe, RNA of PCR-quality was obtained routinely.

Levels of urinary albumin, u-podocyte counts, and nephrin RT-QPCR in pediatric nephrology cases were compared (Table 2). Elevated albuminuria was seen in all cases with abnormal u-podocyte counts (Table 2). In contrast, a large number of subjects with abnormal nephrin RT-QPCR did not have either elevated albuminuria or abnormal u-podocyte counts (samples with abnormal high u-podocyte counts comprised a subset of the abnormal elevated albuminuria samples). These analyses demonstrated that in pediatric nephrology disorders, abnormalities of nephrin RT-QPCR can exist in the absence of u-podocyte abnormalities or elevated albuminuria. Thus, these nephrin RT-QPCR abnormalities are present in a group of patients who would not be identified by other urine-based tests. The absence of elevated albuminuria in subjects with appreciable nephrin RT-QPCR is consistent with such samples coming from early affected subjects who have not yet developed elevated albuminuria.

**RT-QPCR anomalies for nephrin are specific for glomerular disorders.** Table 3 demonstrates the specificity of nephrin RT-QPCR anomalies for glomerular disorders. In many of the examined pediatric glomerular disorders, at least one of the urine sediments showed an abnormality of nephrin RT-QPCR. This is illustrated in Table 3, showing a number of glomerular pediatric nephrology disorders associated with nephrin RT-QPCR in the urine sediment. In contrast, none of the non-glomerular disorders examined showed presence of nephrin in the urine sediment. As expected, nephrin RT-QPCR did not depend on transplant status. One of these abnormalities, cystinosis, showed proteinuria, but did not show an anomaly of nephrin. This is consistent with the tubular rather than glomerular origin of proteinuria in this disorder and demonstrates that abnormal protein in the urine does not *per se* cause a nephrin RT-QPCR anomaly.

**Comparisons over time of analyses of kidney damage in diabetes.** The time for onset of disease can be determined with certainty in type 1 diabetes. In type 1 diabetes, the onset of disease is clear-cut. Further, in type 1 diabetes, the kidney disorder takes many years to develop to the point where it is clinically apparent. Thus, a time line can be drawn by determination of when abnormalities occur after the onset of this disease. A similar analysis can be done for type 2 diabetes although ambiguities inherent in knowing the exact time of onset of type 2 diabetes yield a result that is less clearcut.

Table 4 illustrates that the RT-QPCR anomalies for nephrin are seen in the first 10 years after diagnosis of diabetes more frequently than elevated albuminuria is seen during that interval. This underscores that the nephrin RT-QPCR test is more sensitive than looking for albuminuria or u-podocytes early after diagnosis. Combining the data from Tables 4-6 together demonstrates that RT-QPCR anomalies for nephrin become apparent earlier in the course of disease than the occurrence of elevated albuminuria. The blood pressure of many of the diabetics with abnormalities of nephrin RT-QPCR was normal, so that the abnormalities in Tables 4-6 are not due merely to hypertension (Table 7).

Elevated albuminuria is not present in many urine sediments from individuals showing RT-QPCR anomalies early in the course of disease (Tables 2-8). This makes RT-QPCR valuable as a marker for renal injury as nephrin RT-QPCR should be specific for podocytes and capable of sensitive detection early in the course of disease when proteinuria has not occurred. The difference is that nephrin RT-QPCR assays the current status of the podocytes whereas elevated albuminuria measures the effects of loss of these cells. In sum, the temporal sequence of events in diabetes type 1 is abnormal nephrin RT-QPCR, proteinuria and overt kidney disease.

Electron microscopy demonstrates that there can still be podocytes extant later in the course of disease when nephrin RT-QPCR anomalies are not discernible. There are several non-exclusive possibilities that could underlie this finding:

1. The podocytes that detach may de-differentiate and no longer express nephrin RNA. Such a deficit in the expression of glepp1 has been shown for some podocytes that de-differentiate due to disease.

2. No podocytes that detach at this later time may remain sufficiently viable to contain RNA of good enough quality to support RT-QPCR. Although β-actin analysis is routinely performed to ensure that the urine sediment RNA is sufficiently intact to support RT-QPCR, this cannot guarantee that the RNA of podocytes is sufficiently intact to support RT-QPCR.

These two possibilities are unlikely given the finding of numerous individuals late in the course of disease who have measurable nephrin RT-QPCR.

3. Most likely, therapy is effectively preventing continued loss of podocytes. As a result, nephrin RT-QPCR is negligible. Presumably, the elevated albumin in the urine occurs as a result of older podocyte loss that is beyond the abilities of the kidney to repair. Indeed, when more than *ca.* 50% of podocytes are lost, flattening of the remaining podocytes is insufficient to compensate for previous loss and permanently elevated albuminuria ensues. Thus elevated albuminuria can be observed in the absence of podocyte loss, so that nephrin RT-QPCR can be negligible in such cases where continued loss of podocytes has been staunched.

Supporting the third possibility is the history demonstrating that all of the individuals who had normal nephrin RT-QPCR with elevated albuminuria were on medications to lower blood pressure and decrease podocyte damage. Table 5 illustrates that late in the course of disease, albuminuria can be fixedly abnormal but nephrin RT-QPCR retains the ability to discriminate podocyturia. We examined 18 cases of type 1 diabetes with marked albuminuria who were on therapy (ACE inhibitors or angiotensin 2 receptor blockers). In 8 of these cases, nephrin RT-QPCR was normally low, indicating that the drug therapy was effectively blocking podocyte loss. In the remaining 10 cases, nephrin RT-QPCR anomalies were still seen, indicating that drug therapy was not effectively blocking podocyte loss. In type 2 diabetes, we saw the same phenomenon: 4 out of 8 albuminuric subjects on medication showed a normal low value of nephrin RT-QPCR. This data demonstrates how nephrin RT-QPCR functions to determine the effectiveness of drug therapy. Those cases with low nephrin RT-QPCR indicate cases where the medication is therapeutically inhibiting further loss of podocytes. In accord with the hypothesis that treatment is preventing further loss of podocytes, nephrin mRNA may not be detected in urine sediment cells at this time even though electron microscopy indicates that podocytes remain extant. These cases with low nephrin RT-QPCR indicate cases where the medication is therapeutically inhibiting further loss of podocytes.

The time of onset is more difficult to ascertain in type 2 diabetes than type 1 diabetes, which may explain why the demarcation over time between nephrin RT-QPCR and elevated albuminuria is not as great in type 2 diabetes compared with type 1 diabetes. Nephrin RT-QPCR anomalies are seen in a greater fraction of individuals than go on to develop end stage renal disease (ESRD), underscoring the high prevalence of sub-clinical renal disease by this sensitive analysis in both type 1 and type 2 diabetes.

Longitudinal studies (Table 6) indicate that renal involvement in diabetes may be more common than previously recognized. Over half of the individuals examined had at least one urine sediment nephrin RT-QPCR that was abnormal (nephrin RT-QPCR > 5E+00). Since all the urine sediments were positive for the constitutively expressed β-actin probe, it is likely that the normal samples (nephrin RT-QPCR ≤ 5E+00) indeed reflect an absence of podocyturia. If so, the data in Table 6 indicates that podocyturia is an intermittent and common phenomenon in diabetes. If so, it will be necessary to monitor RT-QPCR of urine sediment longitudinally over the course of disease to monitor the status of the kidneys and response to treatment.

There are thus at least two likely clinical uses of the nephrin RT-QPCR test. Early in the course of disease, nephrin RT-QPCR becomes abnormal before other tests, affording the potential to detect early kidney disease sensitively. Later in the course of treated disease, nephrin RT-QPCR can be normal when elevated albuminuria is present, reflecting the fact that loss of podocytes need not be the permanent type of disorder that elevated albuminuria can become. As a result, nephrin RT-QPCR should be able to determine whether therapy is effectively treating podocyte loss late in the course of disease when elevated albuminuria can be permanently abnormal.

**Hypertension and systemic lupus erythematosus.** In both of these disorders, anomalies of podocytes can be seen (Tables 7-8). Here again, ESRD pathology likely ensues from this podocyte loss. The presence of a significant number of individuals with urine sediment RT-QPCR anomalies for nephrin validates the important role that the podocyte can be expected to play in these disorders.

**Renal cancers.** Cancer is associated with the proliferation of cells that do not adhere normally. As a result, renal cancer may result in the exfoliation of kidney cells not seen in normal urine sediments. Twenty-two such urine sediments that were preserved with RNAlater were obtained from Dr. Pierre Oudet, Strasbourg, France. In 21/22 such sediments, abnormal presence of nephrin was observed. These results indicate that podocyturia and/ or de-differentiation of renal cells to yield expression of nephrin can be used to detect renal cancers. Thus the invention comprises a novel and valuable method both to diagnose renal cancers and to monitor their response to therapy by non-invasive testing of the urine sediment for nephrin transcription. Once again, the sensitive detection of a podocyte-specific transcript is required.

**Nephrin and proteinuria are not correlated.** No correlation was seen between nephrin RT-QPCR and proteinuria (measured by total protein/ creatinine or albumin/creatinine in urine). None of these variables is positive in normal samples, so that all these positive values indicate an anomaly. This lack of correlation demonstrates that the two techniques are measuring different attributes. Nephrin RT-QPCR evaluates the current loss of podocytes by determining the presence of a podocyte-specific gene, nephrin, in urine sediment. It is not surprising that the RT-QPCR test can be abnormal before proteinuria is seen as significant podocyte loss must occur before proteinuria is detectable. Samples that continue to show protein loss in the urine when nephrin RT-QPCR is not appreciable presumably represent individuals who are no longer losing podocytes, but who have significant defects remaining from previous podocyte loss. As a result, there is permanent communication between the blood and urine streams resulting in chronic proteinuria even in the absence of continued podocyte loss.

The nephrin RT-QPCR of some embodiments accomplishes the non-invasive monitoring of disease affecting podocytes. Nephrin RT-QPCR is designed to screen for podocyte disease as it focuses on a gene expressed solely in podocytes in the urinary tract. This intermittent nature of the nephrin RT-QPCR test is reminiscent of the elevated albuminuria test, where the finding of elevated albuminuria is not constant over time. Indeed, elevated albuminuria can regress to normal in diabetic subjects studied longitudinally. These findings are consistent with the hypothesis that the majority of individuals with type 1 diabetes have renal damage from their disease and only a portion of these go on to develop ESRD. Nevertheless, the assay for elevated albuminuria remains useful as individuals with persistently elevated albuminuria are at increased risk to develop nephropathy. Combining the nephrin RT-QPCR and albuminuria tests may permit more exact diagnosis of the stage that the kidney and its treatment are at.

In sum, the nephrin RT-QPCR test (or its analogue) of the invention should be useful both to detect the early effects of diseases affecting podocytes and to monitor the response of podocytes to therapy.

Tables 12-13 illustrate that nephrin RT-QPCR and RT-QPCR for Indian hedgehog another marker for kidney conditions, expressed in proximal tubular cells are often discordant at a given time in a variety of known glomerular disorders (diabetes, hypertension, lupus and glomerular disorders in pediatric nephrology subjects). To study the time course of these abnormalities, the chronic disorder, diabetes, was utilized because the onset is acute and especially easy to document in type 1 diabetes. However, the kidney disorder in diabetes takes many years to develop to the point where it is obvious clinically. Table 12 illustrates that the RT-QPCR anomalies for both nephrin and Indian hedgehog can be seen in the first 10 years after diagnosis more frequently than elevated albuminuria. However, there is discordance, even in the same patient, between the occurrence of podocyte and proximal tubular anomalies (Table 13A). There is also discordance between the RT-QPCR anomalies for Indian hedgehog and elevated albuminuria (Table 13B). Putting the data from Tables 12-13 together demonstrates that the RT-QPCR anomalies for nephrin and/or Indian hedgehog become abnormal earlier in the course of disease than the occurrence of elevated albuminuria. The blood pressure of many of the diabetics with abnormalities of RT-QPCR was normal, so that the abnormalities in Tables 12-13 are not due merely to hypertension (Table 7).

As with the finding that nephrin RT-QPCR can be normal with elevated albuminuria, Indian hedgehog/β-actin RT-QPCR can also be normal with elevated albuminuria. Once again, elevated albuminuria measures damage done over time to the integrity of the podocyte barrier between the blood and urine streams whereas Indian hedgehog/β-actin RT-QPCR measures acute loss of proximal tubular cells.

Sufficient numbers of analyses in diabetes type 1 were performed to examine whether nephrin RT-QPCR, RT-QPCR for Indian hedgehog or proteinuria (measured by total protein/ creatinine or albumin/ creatinine) were correlated. No correlations were seen, so that these variables were not dependent. Note that none of these variables is positive in normals, so that all these positive values indicate an anomaly. It is striking that the RT-QPCR measurements of nephrin and Indian hedgehog were independent of each other in all the disorders studied (Table 9). It is also notable that longitudinal studies of the same individual demonstrated that a given individual can evidence different abnormalities over time (data not shown). Consistent with the finding that elevated albuminuria can vary over time in the same individual, this underscores the intricacies of using urine-based tests to follow the effects on the kidney of diabetes, hypertension and lupus. However, it is clear that the urine-based tests afford a uniquely powerful and non-invasive insight into the effects on the kidney of these disorders. Proximal tubule defects have been suggested in early diabetes as well as focal segmental glomerulosclerosis, and Table 9 provides direct evidence for their existence.

In sum, the invention comprises novel methods, systems and compositions to detect podocyte anomalies of the kidney by examination of urine sediment. In some embodiments, detection of podocytes is accomplished by screening for the transcription of a gene transcribed uniquely by podocytes. The invention comprises the unexpected discovery that desquamation of podocytes occurred in a variety of disorders affecting these cells but did not occur in normals. Further, molecular identification of the cell type desquamated could be accomplished by screening for transcripts from genes selectively expressed in podocytes of urinary tract origin so as to be podocyte-specific genes. These two facts were unknown before the invention. Without limitation, referred embodiments of the invention use nephrin RT-QPCR and Indian hedgehog RT-QPCR to accomplish this task.

The invention comprises screening using RT-QPCR for a podocyte-specific gene (nephrin). The concept of looking for such gene transcripts to identify kidney cells desquamated in the urine is a novel part of the invention. This strategy only became feasible with the ability to preserve urine RNA in urine sediment cells (or fragments of such cells) followed by sensitive analysis of the preserved RNAs. As currently used, in some embodiments, nephrin RT-QPCR will detect podocyte anomalies. Thus, nephrin RT-QPCR represents a useful way of determining the effects of a variety of disorders on podocytes.

### SEQ ID NO: LISTING

SEQ ID NO: 1:
   5'-GCCCCGAGGGTCCTGAAGGTG-3'
SEQ ID NO: 2:
   5'-TGGACTGGCTGACAAAGGGACCCAG-3'
SEQ ID NO: 3:
   5'-TACTGGAGAACCTGAAGACCAGCTGCC-3'
SEQ ID NO: 4:
   5'-CAATTACAATCCAGACATCATCTTCAA-3'
SEQ ID NO: 5:
   5'-CGAGATAGCCAGCGAGTTCAG-3'
SEQ ID NO: 6:
   5'-CATGACCCAGCGCTGCAAGGAC-3'
SEQ ID NO: 7:
   5'-AACTTGAGATGTATGAAGGCTTTTGG-3'
SEQ ID NO: 8:
   5'-TTTTTTTTTTTTTTTTTTTAAG-3'
SEQ ID NO: 9:
   5'-CAACTGGTCTCAAGTCAGTGTACAGGTAAGCCCT-3'

**Table 1. Intra-plate and inter-plate variability of nephrin RT-QPCR**

| | **avg** | **st. dev.** | **# samples** | **p value (plate 1 vs. plate 2)** |
|---|---|---|---|---|
| **pilate 1** | | | | |
| **normal 1** | 4.6E-01 | 2.0E+00 | 19 | **p = 0.352 (not significant)** |
| **normal 2** | 2.5E-01 | 8.8E-01 | 20 | **p = 0.285 (not significant)** |
| | | | | |
| **abnormal 1** | **1.9E+03** | 7.5E+02 | 17 | **p = 0.601 (not significant)** |
| | | | | |
| **plate 2** | | | | |
| **normal 1** | 0.0E+00 | 0.0E+00 | 17 | |
| **normal 2** | 0.0E+00 | 0.0E+00 | 15 | |
| | | | | |
| **abnormal 1** | **2.1 E+03** | 3.9E+02 | 16 | |
| **abnormal 1 dilute 1/10** | **2.5E+02** | 8.8E+01 | 16 | |
| | | | | |
| **all normal samples < 2E00** | | | | |

**Table 2. Comparing nephrin RT-QPCR, u-podocytes & microalbumin/ creatinine in pediatric nephrology samples**

| | **u-podocytes** | **u-podocytes** | **ma/cr <10** | **ma/cr >10** |
|---|---|---|---|---|
| | **negative (<0.6)** | **positive (> 0.6)** | | |
| **RT-QPCR <2** | 18 | 2 | 13 | 7 |
| **RT-QPCR >2** | 10 | 11 | 7 | 14 |
| | | | | |
| **u-podocytes negative (<0.6)** | | | 9 | 19 |
| **u-podocytes positive (> 0.6)** | | | 0 | 13 |

**Table 3. Nephrin is seen in urine sediment only in glomerular disorders**

| **Sample ID** | **Nephrin RT-QPCR** | **urine protein/ creatinine** | **Diagnosis** |
|---|---|---|---|
| **N-711** | **1.2E+02** | **6.68** | **Nephrotic syndrome** |
| | | | |
| **N-712** | **6.6E+03** | **7.01** | **Focal segmental glomerulosclerosis** |
| | | | |
| **N-724** | **9.0E+01** | **0.54** | **Diffuse mesangial proliferative glomerulonephritis** |
| | | | |
| **N-725** | **4.6E+01** | **9.37** | **Focal segmental glomerulosclerosis, s/p transplant** |
| | | | |
| **N-729** | **3.9E+01** | **0.78** | **Focal segmental glomerulosclerosis, s/p transplant** |
| **N-739** | **0.0E+00** | **0.95** | |
| **N-765** | **9.3E+00** | **0.2** | |
| | | | |
| **N-756** | 0.0E+00 | negative | **Chronic glomerulonephritis, s/p transplant** |
| | | | |
| **N-760** | **5.4E+00** | **4.18** | **Acute glomerulonephritis** |
| | | | |
| **N-761** | **1.3E+01** | **5.25** | **Diffuse proliferative glomerulonephritis** |
| | | | |
| **N-766** | **1.4E+04** | **1.62** | **Focal proliferative glomerulonephritis** |
| | | | |
| **N-763** | 0.0E+00 | negative | Dysplasia, s/p transplant |
| **N-767** | 0.0E+00 | negative | Schimke's, s/p transplant |
| **N-726** | 0.0E+00 | negative | Obstructive uropathy, s/p transplant |
| | | | |
| **N-727** | 0.0E+00 | negative | Obstructive uropathy, s/p transplant |
| **N-762** | 0.0E+00 | | |
| **N-728** | 0.0E+00 | negative | Obstructive uropathy, s/p transplant |
| **N-738** | 0.0E+00 | trace | |
| **N-757** | 0.0E+00 | negative | |
| **N-764** | 0.0E+00 | negative | |
| | | | |
| **N-731** | 0.0E+00 | N/A | Cortical necrosis, s/p transplant |
| **N-743** | 0.0E+00 | negative | |
| **N-759** | 4.1 E+00 | negative | |
| | | | |
| **N-736** | 0.0E+00 | trace | Juvenile Nephronophthisis, s/p transplant |
| | | | |
| **N-737** | 0.0E+00 | negative | Dysplasia, s/p transplant |
| | | | |
| **N-740** | 0.0E+00 | **3+** | Cystinosis, s/p transplant |
| **N-758** | 1.4E+00 | trace | |

**Table 4. Time course of nephrin RT-QPCR and albuminuria abnormalities in diabetes**

| **Type 1 diabetes** | **nephrin RT-QPCR** | | **abnormal urine albumin/ creatinine** | |
|---|---|---|---|---|
| **0-10 yrs** | 28.3% | (15/53) | 10.6% | (5/47) |
| **11-48yrs** | 28.9% | (11/38) | 47.2% | (17/36) |
| **0-48 yrs** | 28.6% | (26/91) | 23.70% | (22/83) |
| | | | | |

| **Type 2 diabetes** | **nephrin RT-QPCR** | | **abnormal urine albumin/ creatinine** | |
|---|---|---|---|---|
| **0-10 yrs** | 45.5% | (5/11) | 23.1% | (3/13) |
| **11-40 yrs** | 45.5% | (5/11) | 41.7% | (5/12) |
| **0-40 yrs** | 45.5% | (10/22) | 32.0% | (8/25) |

**Table 5. Relationship between nephrin RT-QPCR and albuminuria abnormalities in type 1 diabetes**

| | **nephrin RT-QPCR** | **urinary albumin/ creatinine** | **urinary albumin/ creatinine** |
|---|---|---|---|
| | | **<30** | **>30** |
| **nephrin-** | **0-5** | 60 | 9 |
| **nephrin+** | **>5** | 36 | 7 |

**Table 6. Type I diabetes longitudinal analysis**

| **Each cluster represents longitudinal urine sediment samples from a single individual with type I diabetes** | | | |
|---|---|---|---|
| | **no podocyturia Nephrin RT-QPCR** | | **podocyturia RT-QPCR nephrin** |
| D84 | 0.0E+00 | D131 | 0.0E+00 |
| D121 | 0.0E+00 | D220 | **9.3E+01** |
| D350 | 0.0E+00 | D404 | **7.0E+01** |
| D592 | 0.0E+00 | D574 | **3.5E+02** |
| | | | |
| D250 | 0.0E+00 | D106 | **1.9E+02** |
| D517 | 2.8E+00 | D167 | 0.0E+00 |
| D727 | 0.0E+00 | D495 | **2.3E+02** |
| | | D724 | 0.0E+00 |
| | | | |
| D310 | 0.0E+00 | D344 | 2.0E+00 |
| D347 | 1.2E+00 | D492 | **1.3E+01** |
| D621 | 0.0E+00 | D553 | 0.0E+00 |
| | | | |
| D155 | 0.0E+00 | D242 | **4.0E+02** |
| D266 | 0.0E+00 | D430 | 4.5E+00 |
| D529 | 2.7E+00 | D566 | 0.0E+00 |
| | | | |
| D215 | 0.0E+00 | D122 | **3.6E+02** |
| D436 | 0.0E+00 | D325 | 0.0E+00 |
| D643 | 0.0E+00 | D561 | 4.8E+00 |
| | | | |
| | | D218 | **3.6E+01** |
| | | D239 | 0.0E+00 |
| | | D300 | 0.0E+00 |
| | | | |
| | | D129 | 0.0E+00 |
| | | D235 | **1.1 E+02** |
| | | D603 | 0.0E+00 |

**Table 7. Hypertension**

| **Hypertension** Sample # | **nephrin RT-QPCR** | **Total protein/ creatinine** |
|---|---|---|
| 128 | 0.0E+00 | |
| 129 | 0.0E+00 | |
| 130 | 0.0E+00 | |
| 131 | 0.0E+00 | |
| 132 | 0.0E+00 | |
| 136 | 0.0E+00 | |
| 138 | 0.0E+00 | |
| 139 | 0.0E+00 | |
| 140 | 0.0E+00 | |
| 141 | 0.0E+00 | |
| 142 | 0.0E+00 | |
| 144 | 0.0E+00 | |
| 146 | 0.0E+00 | |
| 147 | 0.0E+00 | |
| 149 | 0.0E+00 | |
| 154 | 0.0E+00 | 0.16 |
| 155 | 0.0E+00 | 0.06 |
| 157 | 0.0E+00 | 0.07 |
| 158 | 0.0E+00 | **1.09** |
| 163 | 0.0E+00 | 0.09 |
| 166 | 0.0E+00 | 0.09 |
| 167 | 0.0E+00 | |
| 168 | 0.0E+00 | 0.13 |
| 169 | 0.0E+00 | 0.08 |
| 161 | 0.0E+00 | 0.10 |
| 160 | 1.1E+00 | 0.03 |
| 145 | 0.0E+00 | |
| 159 | 1.7E+00 | 0.10 |
| 148 | 2.3E+00 | |
| 162 | 3.0E+00 | 0.08 |
| 156 | 4.3E+00 | |
| 135 | 9.0E+00 | |
| | | |
| 143 | **1.0E+01** | |
| 134 | **2.6E+01** | |
| 150 | **7.8E+01** | |
| 133 | **9.5E+01** | |
| 151 | **2.0E+02** | |
| 137 | **4.5E+02** | |
| 152 | **1.4E+03** | |
| 164 | **1.9E+03** | 0.06 |
| 153 | **2.3E+03** | 0.06 |
| 165 | **1.8E+04** | 0.05 |
| abnormal values are in bold face | | |

**Table 8. Systemic lupus erythematosus (SLE)**

| **sample** | **nephrin RT-QPCR** | **Protein/ Creatinine** |
|---|---|---|
| SLE12 | 0.0E+00 | 0.17 |
| SLE19 | 0.0E+00 | |
| SLE21 | 0.0E+00 | |
| SLE24 | 0.0E+00 | negative |
| SLE25 | 0.0E+00 | |
| SLE26 | 0.0E+00 | |
| SLE28 | 0.0E+00 | negative |
| SLE29 | 0.0E+00 | negative |
| SLE30 | 0.0E+00 | 0.16 |
| SLE31 | 0.0E+00 | negative |
| SLE32 | 0.0E+00 | |
| SLE33 | 0.0E+00 | |
| SLE34 | 0.0E+00 | |
| SLE35 | 0.0E+00 | |
| SLE15 | 1.2E+00 | 1.11 |
| SLE27 | 1.3E+00 | |
| SLE23 | 1.7E+00 | |
| SLE22 | 0.0E+00 | |
| SLE18 | 3.8E+00 | |
| SLE11 | 5.4E+00 | **2.65** |
| SLE17 | 6.2E+00 | |
| SLE13 | **4.0E+01** | |
| SLE16 | **4.5E+01** | |
| SLE14 | **7.4E+01** | |
| SLE20 | **7.6E+01** | |

**Table 9. Indian hedgehog and nephrin mRNA presence in urine sediment in different conditions.**

| | **Indian hedgehog / nephrin** | | | |
|---|---|---|---|---|
| | **positive/positive** | **positive/negative** | **negative/positive** | **negative/negative** |
| **Normal controls** | 0 | 0 | 0 | 40 |
| | | | | |
| **Diabetes** | 3 | 21 | 20 | 51 |
| **Hypertension** | 1 | 6 | 7 | 15 |
| **Lupus** | 2 | 1 | 2 | 9 |
| | | | | |
| **Pediatric nephrology** | | | | |
| **Obstruction** | 1 | 10 | 0 | 12 |
| **Non-obstructive disease** | 2 | 11 | 6 | 18 |

**Table 10. Indian hedgehog can be abnormally present in urine sediment in obstruction**

| (abnormal values > 1E-04 are in bold face) | | | |
|---|---|---|---|
| **10A.** | **Individual cases** | | |
| **Sample** | | **IHH/actin** | Diagnosis |
| | 1 | 0.0E+00 | posterior urethral valves, s/p multiple reconstructions |
| | 2 | 0.0E+00 | s/p transplant (father), obstructive uropathy |
| | 3 | 0.0E+00 | Bilateral grade IV vesicoureteral reflux, s/p reimplantation, Duplicated left renal collecting system, Pyelonephritis, diagnosed in 1994, nl u/a, mild familial hearing loss |
| | 4 | 0.0E+00 | Chronic renal failure secondary to obstructive uropathy, Vesicoureteral reflux, bilateral status post left nephrectomy and reimplantation, Bladder dysfunction, Right megaureter, Hyperparathyroidism secondary to chronic renal failure, u/a 2+ wbc, tr rbc, |
| | 5 | 0.0E+00 | ESRD secondary to obstructive uropathy, u/a tr blood |
| | 6 | 0.0E+00 | History of posterior urethral valves, status post resection |
| | 7 | 3.6E-05 | renal failure secondary to obstructive prune belly |
| | 8 | **2.4E-03** | sacral agenesis and neurogenic bladder dysfunction; solitary kidney with obstructive symptoms |
| | 9 | **6.2E-03** | chronic renal failure secondary to obstructive uropathy |
| | 10 | **9.5E-03** | Posterior urethral valve with obstructive uropathy., Status post renal transplant March 1995, living related from the father., u/a nl |
| | 11 | **4.2E-02** | grade 2 reflux, pyelonephritis in May |
| | 12 | **4.8E-02** | pyelonephritis, obstructive uropathy, chronic renal failure |
| | 13 | **1.5E-01** | posterior urethral valves, hypertension |
| | 14 | **1.1E-01** | s/p urethral vavles, surgery 5/26/98 |
| | 15 | **5.9E-01** | end-stage renal disease secondary to obstructive, uropathy, primarily neurogenic bladder who received and cadaveric renal transplant 2 weeks ago, u/a 3+ blood, tr protein |

| **10B. Cases followed longitudinally** | | | |
|---|---|---|---|
| **sample** | **Date of sample** | **IHH/actin** | **Diagnosis** |
| Patient 1 | 08/08/00 | **1.1 E-01** | Chronic renal insufficiency; obstructive uropathy; pericardial effusion; renal hypertension |
| | 11/03/00 | 0.0E+00 | |
| | 12/26/00 | **3.8E-02** | |
| | 01/12/01 | **2.3E-02** | |
| | 05/29/01 | 0.0E+00 | |
| Patient 2 | 01/09/01 | **3.3E-02** | Renal failure secondary to obstructive uropathy, status post cadaveric renal transplant |
| | | | |
| | 01/16/01 | **2.3E-02** | |
| | 01/23/01 | **4.3E-03** | |
| | 01/30/01 | 0.0E+00 | |
| | 02/06/01 | **3.1 E-01** | |
| | 02/20/01 | 0.0E+00 | |
| | 03/13/01 | **5.3E-02** | |
| | 04/03/01 | **1.1 E-02** | |
| | 04/16/01 | **0.0E+00** | |
| | 05/12/01 | 0.0E+00 | |
| | 06/05/01 | 0.0E+00 | |
| | | | |
| Patient 3 | 07/18/00 | 0.0E+00 | posterior urethral valves, transplanted |
| | 07/25/00 | **9.6E-02** | |
| | 08/01/00 | 0.0E+00 | |
| | 08/08/00 | 0.0E+00 | |
| | 08/15/00 | 0.0E+00 | |
| | 08/29/00 | **4.2E-01** | |
| | 09/12/00 | **6.4E-02** | |
| | 10/03/00 | **2.0E-01** | |
| | 10/17/00 | **2.1E-01** | |
| | 12/21/00 | **1.3E-01** | |
| | 01/12/01 | **5.3E-03** | |
| | 01/25/01 | **1.6E-01** | |
| | 03/27/01 | **1.7E-02** | |
| | 06/05/01 | **1.9E-01** | |
| | | | |
| Patient 4 | 07/20/99 | 0.0E+00 | s/p cadaveric transplant 7/93 for ESRD due to obstructive uropathy |
| | | | |
| | 06/05/01 | 0.0E+00 | |
| | 07/10/01 | **8.1E-02** | |
| | 07/31/00 | **6.8E-04** | |
| | | | |
| Patient 5 | 08/15/00 | **7.9E-01** | posterior urethral valve, s/p transplant, not compliant with transplant medication |
| | | | |
| | 11 /14/00 | **3.9E-03** | |
| | 01/16/01 | **6.9E-02** | |
| | 03/13/01 | **1.8E-02** | |
| | 04/15/01 | **1.1E-02** | |
| | 05/08/01 | **6.5E-02** | |

**Table 11. IHH excretion into urine of subjects with acute tubular necrosis**

| **sample** | **RT-QPCR IHH/β actin** | **Hx** |
|---|---|---|
| ATN525 | **2.6E-03** | ATN # 1 (after surgery for liver transplant); non-oliguric |
| ATN526 | **3.8E-03** | ATN #2 |
| ATN527 | **1.5E-03** | ATN # 3 |
| ATN531 | **3.0E-03** | ATN #4 |
| | | |
| ATN528 | **2.3E-03** | Acute renal failure 2 weeks after liver transplant |
| | | |
| ATN529 | 0.0E+00 | non-oliguric renal failure after surgery, expired |
| | | |
| ATN550 | 0.0E+00 | s/p tracheostomy |
| | | |
| ATN551 | 0.0E+00 | s/p abdominal surgery |
| | | |
| A345 | 0.0E+00 | cirrhosis due to alcohol |
| | | |
| A346 | **4.5E-03** | acute renal failure with likely ATN s/p surgery |
| | | |
| A382 | **1.6E-03** | auto accident, s/p surgery, acute renal failure, deceased |
| | | |
| A405 | 0.0E+00 | obese, DM, s/p surgery, acute lack of urine output, deceased |

**Table 12. Time course of nephrin, Indian hedgehog (IHH) and albuminuria abnormalities in diabetes**

| **Diabetes type** I | **nephrin** | | **IHH/actin** | | **albumin/ creatinine** | |
|---|---|---|---|---|---|---|
| **0-10 yrs** | 28.3% | (15/53) | 26.7% | (12/45) | 10.6% | (5/47) |
| **11-48 yrs** | 28.9% | (11/38) | 31.4% | (11/35) | 47.2% | (17/36) |
| **0-48 yrs** | 28.6% | (26/91) | 28.8% | (23/80) | 23.7% | (22/83) |
| | | | | | | |

| **Diabetes type 2** | | | | | | |
|---|---|---|---|---|---|---|
| 0-10 yrs | 45.5% | (5/11) | 30.8% | (4/13) | 23.1% | (3/13) |
| 11-40 yrs | 45.5% | (5/11) | 16.7% | (2/12) | 41.7% | (5/12) |
| 0-40 yrs | 45.5% | (10/22) | 24.0% | (6/25) | 32.0% | (8/25) |

**Table 13. Relationships among nephrin, Indian hedgehog (IHH) and albuminuria abnormalities in diabetes**

| **Table 13A.** | **RT-QPCR nephrin** | **RT-QPCR IHH-** | **RT-QPCR IHH+** |
|---|---|---|---|
| | | <1E-04 | >1E-04 |
| **nephrin-** | 0-5 | 29 | 16 |
| **nephrin+** | >5 | 22 | 4 |
| | | | |

| **Table 13B.** | RT-QPCR | albumin/ creatinine | albumin/ creatinine |
|---|---|---|---|
| | IHH | <30 | >30 |
| **IHH-** | 0-1 E-04 | 33 | 9 |
| **IHH+** | >1E-04 | 16 | 3 |

## Claims

1. A method for detectine a kidney glomerular disorder, comprising the steps of: screening a human urine sample for expression of nephrin gene RNA that is present in said urine sample; and detecting kidney disease based on the presence of nephrin gene RNA in the urine sample.

2. The method of Claim 1, wherein the screening step comprises determining the level of nephrin gene RNA by a reverse transcriptase quantitative polymerase chain reaction assay.

3. The method of Claim 2, wherein the detecting is based on a decrement of fluorescence energy transfer measured from a PCR-mediated disruption of an oligonucleotide duplex modified with donor and acceptor fluorophores.

## Patentansprüche

1. Verfahren zur Bestimmung einer glomerulären Nephropathie umfassend die Schritte: Reihenuntersuchen von Proben mit menschlichem Urin auf die Expression der RNA des Nephrin-Gens, welches in der Urinprobe zugegen ist, und Ermitteln einer Nephropathie auf der Grundlage der Gegenwart von RNA des Nephrin-Gens in der Urinprobe.

2. Verfahren nach Anspruch 1, wobei der Untersuchungsschritt umfasst das Bestimmen des RNA-Spiegels des Nephrin-Gens mittels eines quantitativen RT-PCR-Assays.

3. Verfahren nach Anspruch 2, wobei das Ermitteln erfolgt auf Grundlage der Abnahme des Fluoreszenzenergietranfers, der gemessen wird anhand einer PCR-vermittelten Zerstörung eines Oligonucleotid-Duplexes, welcher mit Donor- und Akzeptor-Fluorophoren modifiziert ist.

## Revendications

1. Procédé pour détecter une maladie glomérulaire des reins, comprenant les étapes d'analyser un échantillon d'urine humaine pour l'expression d'ARN pour le gène de la néphrine qui est présent dans ledit échantillon d'urine ; et détection une maladie rénale sur la base de la présence du l'ARN pour le gène de la néphrine dans l'échantillon d'urine.

2. Procédé selon la revendication 1 dans lequel l'étape d'analyser comprend déterminer le niveau d'ARN pour le gène de la néphrine par une amplification en chaîne par polymérase quantitative par transcriptase inverse.

3. Procédé selon la revendication 2 dans lequel la détection est basée sur un décrément de transfert d'énergie de fluorescence mesuré à partir d'une disruption d'un duplex d'oligonucléotides modifié par des fluorophores donneurs et accepteurs.


### SEQUENCE LISTING

<110> Kurnit, David M
<120> Methods and compositions for Analysis of urine samples in the Diagnosis and Treatment of Kidney Diseases -
<130> 65306-0083
<150> Us 10/108,969
   <151> 2002-03-28
<160> 9
<170> PatentIn version 3.1
<210> 1
   <211> 21
   <212> DNA
   <213> Artificial sequence
<220>
   <223> Human Nephrin Excess Primer
<400> 1
   gccccgaggg tcctgaaggt g 21
<210> 2
   <211> 25
   <212> DNA
   <213> Artificial sequence
<220>
   <223> Amplisensor Probe
<400> 2
   tggactggct gacaaaggga cccag 25
<210> 3
   <211> 27
   <212> DNA
   <213> Artificial Sequence
<220>
   <223> Amplisensor Probe
<400> 3
   tactggagaa cctgaagacc agctgcc 27
<210> 4
   <211> 27
   <212> DNA
   <213> Artificial sequence
<220>
   <223> Indian Hedgehog Reverse Primer
<400> 4
   caattacaat ccagacatca tcttcaa 27
<210> 5
   <211> 21
   <212> DNA
   <213> Artificial sequence
<220>
   <223> Indian Hedgehog Reverse Primer
<400> 5
   cgagatagcc agcgagttca g 21
<210> 6
   <211> 22
   <212> DNA
   <213> Artificial sequence
<220>
   <223> Indian Hedgehog TaqMan Probe
<400> 6
   catgacccag cgctgcaagg ac 22
<210> 7
   <211> 26
   <212> DNA
   <213> Artificial sequence
<220>
   <223> Human Beta-actin Forward Primer
<400> 7
   aacttgagat gtatgaaggc ttttgg 26
<210> 8
   <211> 32
   <212> DNA
   <213> Artificial sequence
<220>
   <223> Human Beta-actin Reverse Primer
<400> 8
   tttttttttt tttttttttt ttttttttta ag 32
<210> 9
   <211> 34
   <212> DNA
   <213> Artificial sequence
<220>
   <223> Human Beta-actin TaqMan Probe
<400> 9
   caactggtct caagtcagtg tacaggtaag ccct 34